# EUROPEAN PATENT APPLICATION

(11) **EP 1 389 446 A2**
(43) Date of publication of application: **18.02.2004**
(21) Application number: 03255902.3
(22) Date of filing: 22.10.1999
(51) Int. Cl.: A61B 17/28

(54) **Surgical grasper with inserts**

(30) Priority: 23.10.1998 US 105406 P; 23.09.1999 US 156032 P
(62) Divisional of application: 99970903.3
(71) Applicant: APPLIED MEDICAL RESOURCES CORPORATION, Laguna Hills, CA 92653 (US)
(72) Inventor: Taylor, Scott, Mission Viejo, California 92692 (US)
(74) Representative: Kensett, John Hinton

(57) **Abstract**

A handle assembly is operable by user through a linkage to open and close first and second jaws, moveable with respect to axis. A ratchet assembly is included in the linkage and comprises a series of ratchet teeth and a ratchet pawl, which cooperate to maintain the jaws in a progressively closed position. A lock coupled to either the ratchet teeth, or the pawl maintains these elements in a spaced relationship to inhibit operation of the ratchet assembly until the lock is manually released. An insert carried by a jaw support includes a distal high-profile pad and a proximal low-profile pad, which may be covered by a woven mesh to enhance traction. An associated method includes the step of manually locking one of the ratchet elements in a spaced relationship with the other of the ratchet elements.

## Description

### Cross-Reference to Related Applications

This application is based on and claims the priority of U.S. provisional application serial no. 60/105,406, filed on October 23, 1998, and entitled "Surgical Grasper with Inserts and Method of Using Same", as well as U.S. provisional application serial no. 60/156,032, filed on September 23, 1999, and entitled "Laparoscopic Grasper with Atraumatic Babcock Jaw Insert."

### Field of the Invention

This invention relates generally to surgical instruments, and more specifically to a laparoscopic grasper having atraumatic jaw inserts.

### Discussion of the Prior Art

Graspers of the prior art have utilized Babcock jaws formed of metal to achieve secure grasping and mobilization of an organ or tissue. The Babcock jaw shape is particularly effective at grasping and capturing a portion of a large organ, such as the stomach, and is almost always used for mobilizing the stomach in a procedure known as laparoscopic Nissen fundoplication. When the Babcock jaws are formed of metal, they provide a high degree of traction, but also tend to traumatize the immediate and surrounding tissue in the process. The metal jaws have also tended to perforate delicate tissue when an excessive clamping force is applied. To address this inadequacy, graspers of the prior art have been designed to minimize or regulate the allowable clamping pressure of the metal jaws. While it is known that trauma to tissue having an irregular shape can be controlled by conforming or deforming the clamping jaws, this has not been possible with the metal jaws of the prior art. '

Graspers of the prior art have included ratcheting mechanisms and have required movement of a lever to temporarily disengage the ratchet mechanism each time the grasper jaws were closed. A procedure requiring the surgeon to "run" the jaws through a large length of tubing, such as the bowel, has been relatively impossible using this type of ratchet mechanism. As a consequence, operating rooms have been required to have both types of graspers, those with and those without ratcheting mechanisms.

### Summary of the Invention

The atraumatic laparoscopic grasper of the present invention overcomes these deficiencies of the prior art. This is accomplished by incorporating a Babcock-shaped, disposable insert into a jaw assembly. With this configuration, the Babcock shape of the insert serves to capture and hold tissue, such as the fundus of the stomach, while the elastomeric pads eliminate tissue trauma. In addition to providing the high degree of atraumatic traction, the elastomeric pads are also capable of conforming and deforming to accommodate tissues of irregular shape. With this configuration, the device is particularly effective at capturing and holding flaccid tissue such as the fundus of the stomach.

The grasper also includes an enhanced insert to jaw attachment system that consists of a pair of vertical notches formed into each jaw, and a corresponding pair of vertical ribs molded into each insert. The ribs on the insert seat in the notches of the jaws and prevent axial removal of the insert from the jaw. The enhanced attachment system allows the device to be used for axially pulling organs or tissues without the possibility of insert slippage or movement in the axial direction.

The laparoscopic grasper also includes a ratcheting mechanism that allows the jaws to be locked in place. The mechanism is normally active so that operation of the handle of the device not only closes the jaws, but maintains the jaws in a closed, locked configuration. To unlock the jaws, a trigger is provided to temporarily deactivate the ratcheting mechanism. A novel thumbscrew can be operated to completely deactivate the ratcheting mechanism. This allows the surgeon to quickly grasp and release tissue without having to repeatedly activate the trigger.

The present invention obviates the need for an operating room to stock two types of graspers, by first including a trigger that can temporarily deactivate the ratchet mechanism, and also including a novel thumbscrew that can completely deactivate the ratchet mechanism.

These and other features and advantages of the present invention will be better understood with a description of preferred embodiments of the invention and reference to the associated drawings.

### Description of the Drawings

Fig. 1 is a perspective view of a laparoscopic grasper, positioned for insertion into a patient to grasp the stomach of the patient;
Fig. 2 is a perspective view of the grasper illustrated in Fig. 1;
Fig. 3 is an enlarged perspective view of the jaws at the distal end of the graspers, the jaws being illustrated in an open position;
Fig. 4 is a side-elevation view of the jaw assembly, with the jaws illustrated in a closed position;
Fig. 5 is a bottom-side perspective view of a jaw insert associated with the jaw assembly;
Fig. 6 is a topside perspective view of one of the jaws associated with the grasper;
Fig. 7 is a side-elevation view illustrating the pivotal mounting of a jaw insert on a jaw of the grasper;
Fig. 8 is an axial cross-section view of the grasper taken along lines 8-8 of Fig. 2 and illustrating a ratchet mechanism at the proximal end of the grasper;
Fig. 9 is an enlarged axial cross-section view of the ratchet mechanism and associated handle assembly;
Fig. 10 is an enlarged axial cross-section view of the ratchet mechanism in an active state;
Fig. 11 is an axial cross-section view of the ratchet mechanism and associated handle assembly;
Fig. 12 is an axial cross-section view of the ratchet mechanism in a temporarily deactivated state;

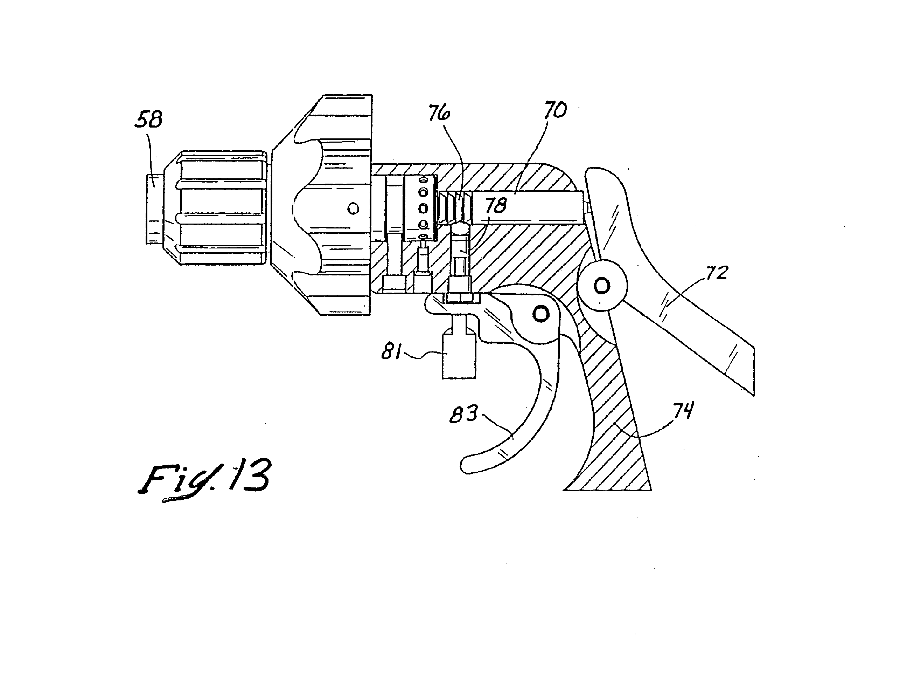

Fig. 13 is an axial cross-section view of the ratchet mechanism in a permanently deactivated state;
Fig. 14 is a side-elevation view of a further embodiment of the grasper of the present invention;
Fig. 15 is a perspective view of the jaw assembly associated with the embodiment of Figure 14;
Fig. 16 is a side-elevation view of the jaw assembly of Figure 15;
Fig. 17 is an axial cross-section view of the jaw assembly of Figure 16;
Fig. 18 is a side-elevation view of the handle assembly and a ratchet assembly disposed in a first position to provide the grasper with ratchet characteristics; and
Fig. 19 is a side-elevation view illustrating the ratchet assembly in a second position inhibiting the ratchet characteristics.

### Description of Preferred Embodiments and Best Mode of the Invention

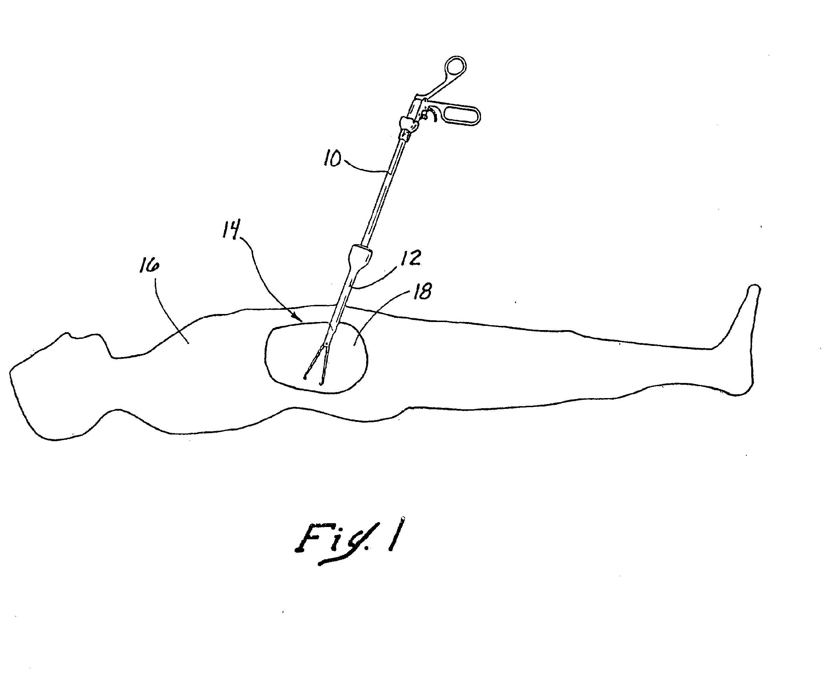

A laparoscopic grasper is illustrated in Figure 1 and designated generally by the reference numeral 10. The grasper 10 is operatively positioned through a trocar 12 and extends into an abdomen 14 of a patient 16. Within the abdomen 14, the grasper 10 can be operated to grasp a stomach 18, for example, and to move the stomach as required by the surgical procedure.

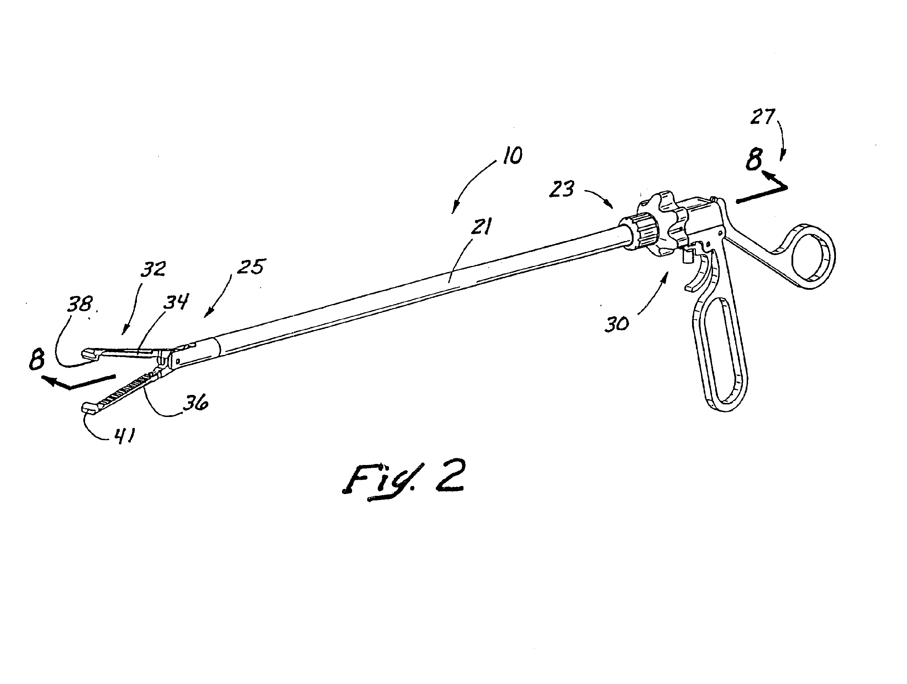

With reference to Figure 2, it can be seen that the grasper 10 of this embodiment includes a barrel 21 extending between a proximal end 23 and a distal end 25. A handle assembly 27 and ratchet mechanism 30 are disposed at the proximal end 23 and movable to operate a jaw assembly 32 at the distal end 25.

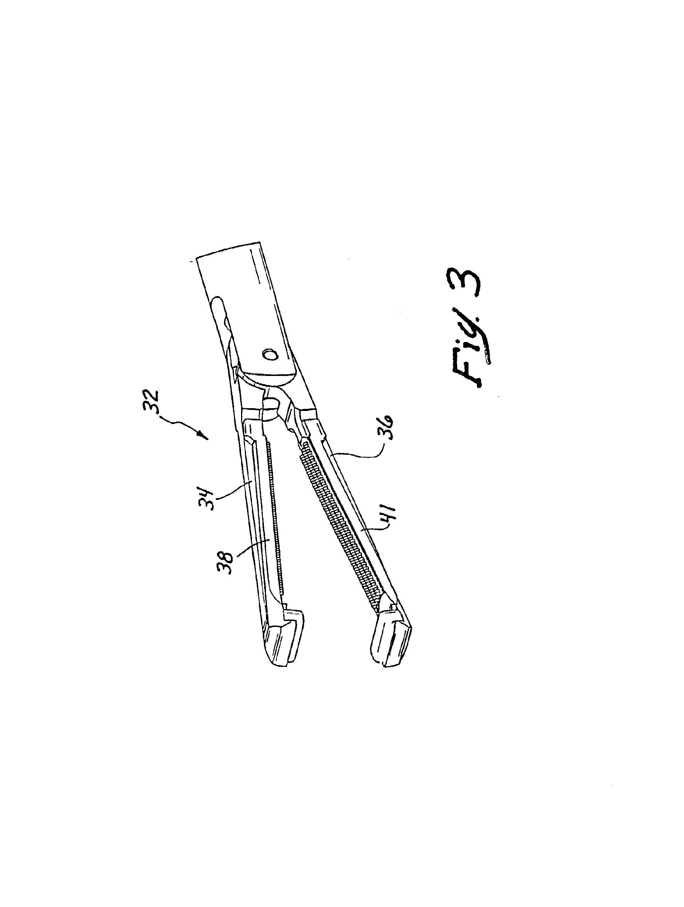

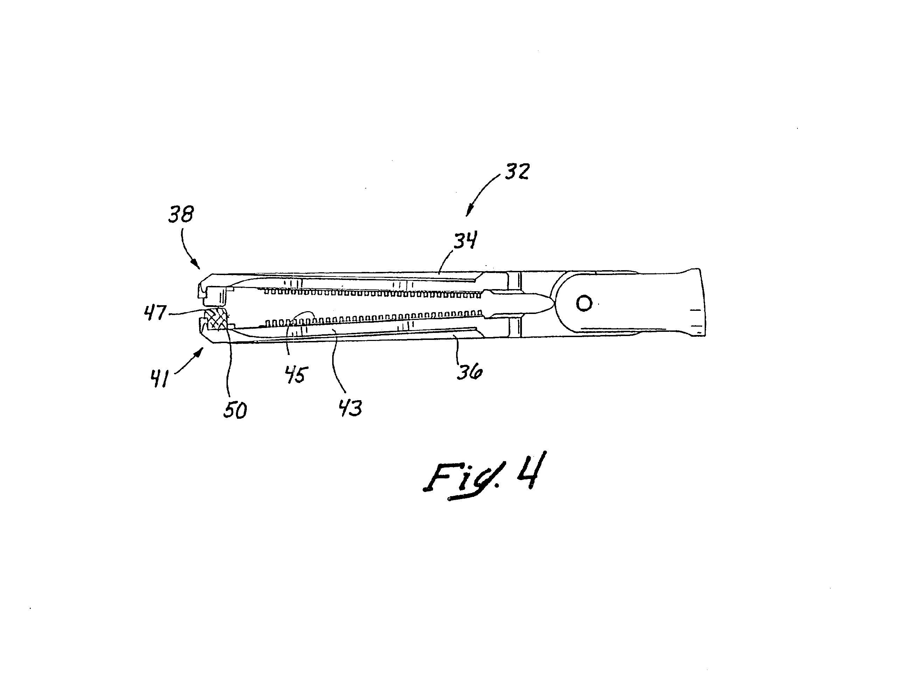

In Figure 3, the jaw assembly 32 is illustrated to include a pair of opposing jaws 34 and 36 relatively movable between an open position, as illustrated in Figure 3, and a closed position, as illustrated in Figure 4. Each of the jaws 34 and 36 is provided with an associated atraumatic insert 38 and 41, respectively.

The insert 41 of this embodiment is best illustrated in Figures 3-5 to include an elongate injection-molded plastic insert base 43, an insert-molded low-profile atraumatic pad 45, and a high-profile pad 47 which may be wrapped with a polyester braid material 50.

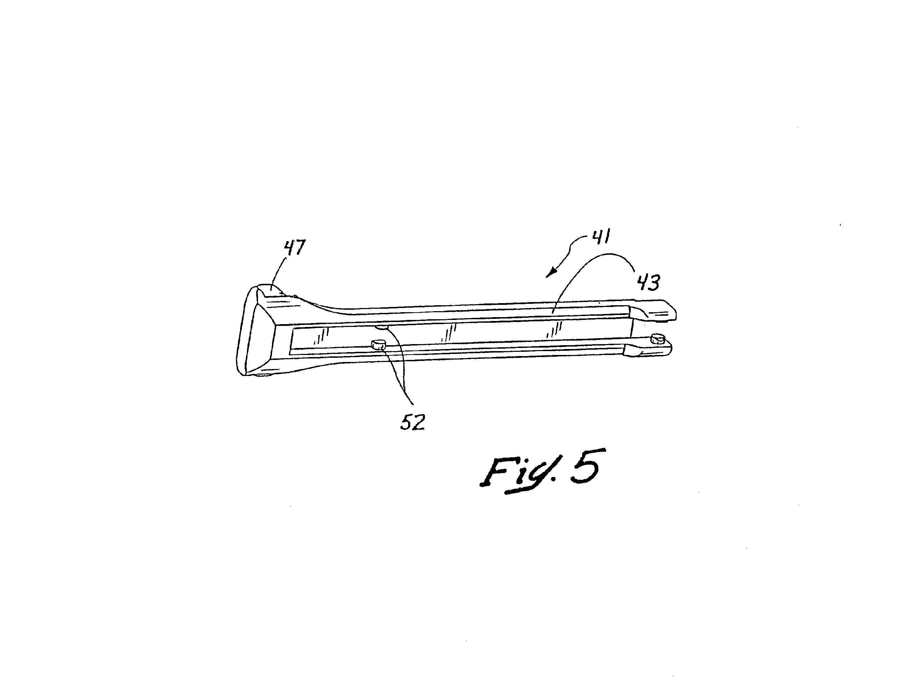

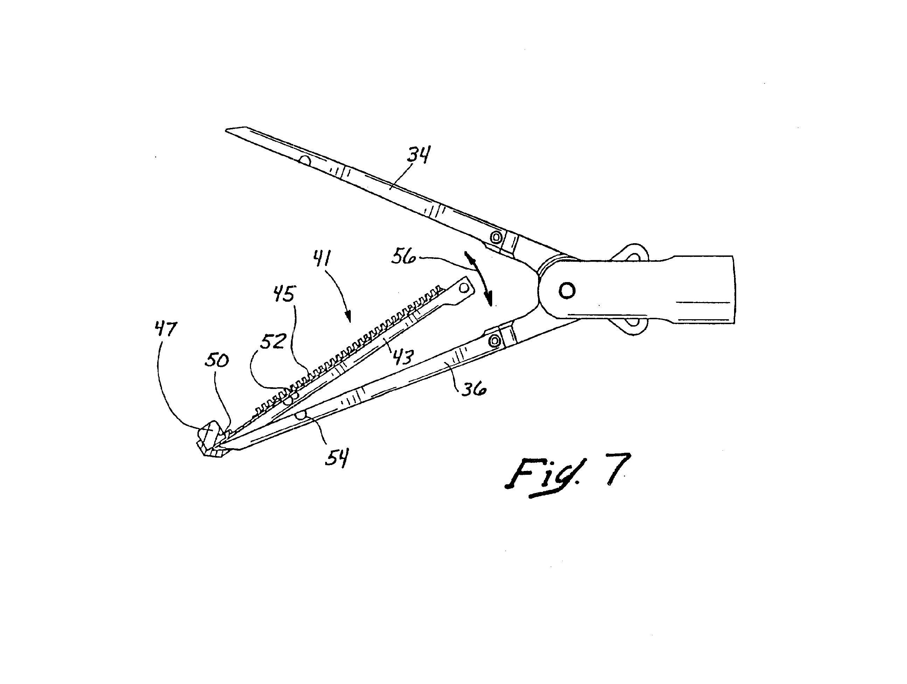

As best illustrated in Figure 5, the disposable insert 41 also incorporates a pair of verticle ribs 52, which are positioned to nest in a corresponding pair of vertical notches 54 in the insert jaw 36. The notches 54 are located near the distal tip of the jaw 36 so that the strength of the jaw is not compromised. In this embodiment, the ribs 52 are preferably an integral portion of the injection-molded, plastic insert base 43. Their function is to prevent the insert from being pulled axially distally relative to the jaw 36 in order to prevent axial removal of the insert from the jaw. With the interaction of the ribs 52 and notches 54, the insert 41 is removable from the jaw 36 only with a pivotal motion, as illustrated by an arrow 56 in Figure 7.

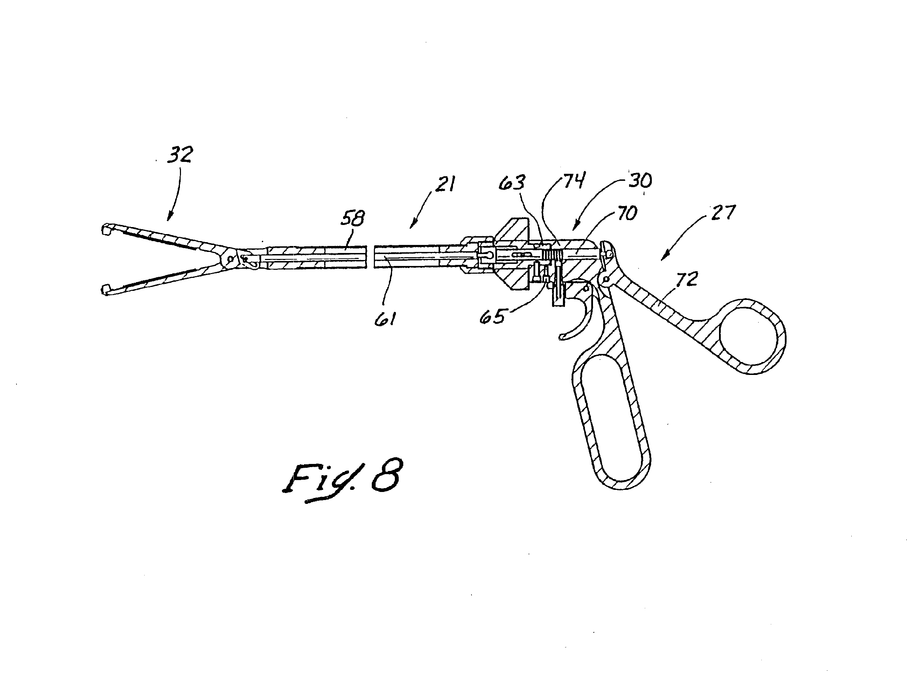

An axial cross-section view of the grasper 10, is illustrated in Figure 8. In this view, it can be seen that the barrel 21 of a preferred embodiment includes an outer tube 58 and a central shaft 61 that are relatively movable to operate the jaw assembly 32. Relative movement of the outer tube 58 and inner shaft 61 is accomplished by operation of the handle assembly 27 and associated ratchet mechanism 30. An apertured cylinder 63 operating in combination with an associated pin 65 provides the grasper 10 with a capability for indexing the jaw assembly 32.

In the illustrated embodiment, an extension 70 is coupled between the inner shaft 61 and a handle 72, which is pivotal on a handle housing 74 fixed to the tube 58. Movement of the handle 72 relative to the handle housing 74 moves the extension 70 and associated shaft 61 relative to the tube 58 and thereby operates the jaw assembly 32.

The ratchet assembly 30 includes a series of ratchet teeth 76. These teeth 76 are formed along the extension 70 and are moveable relative to a pawl 78 which is coupled through the handle housing 74 to a thumbscrew 81. As the extension 70 and associated shaft 61 are moved relative to the tube 58 to close the jaw assembly 32, the ratchet teeth 76 slides over the pawl 78 to lock the jaw assembly 32 in its most closed position.

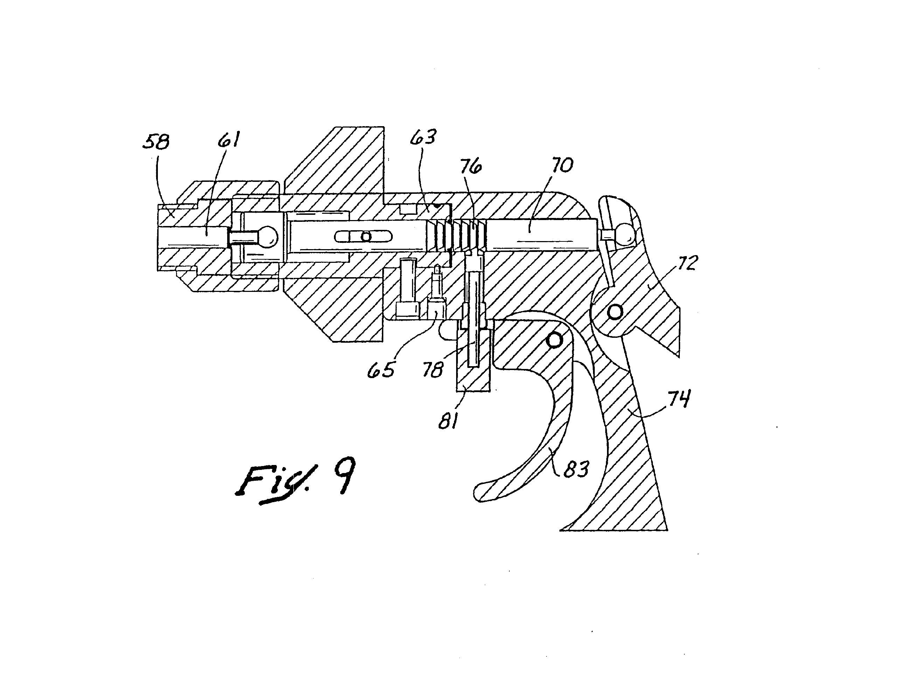

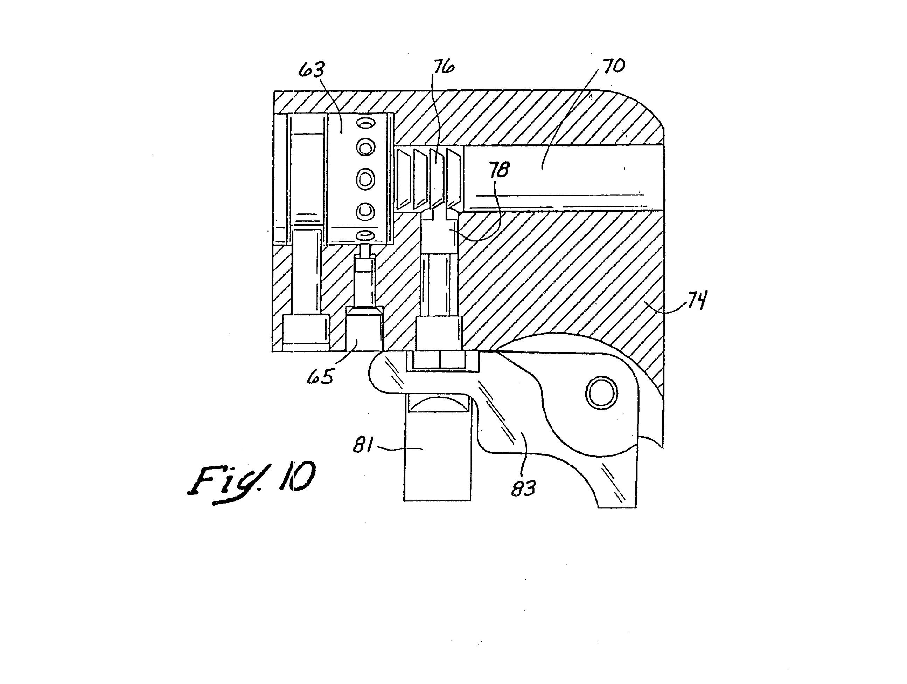

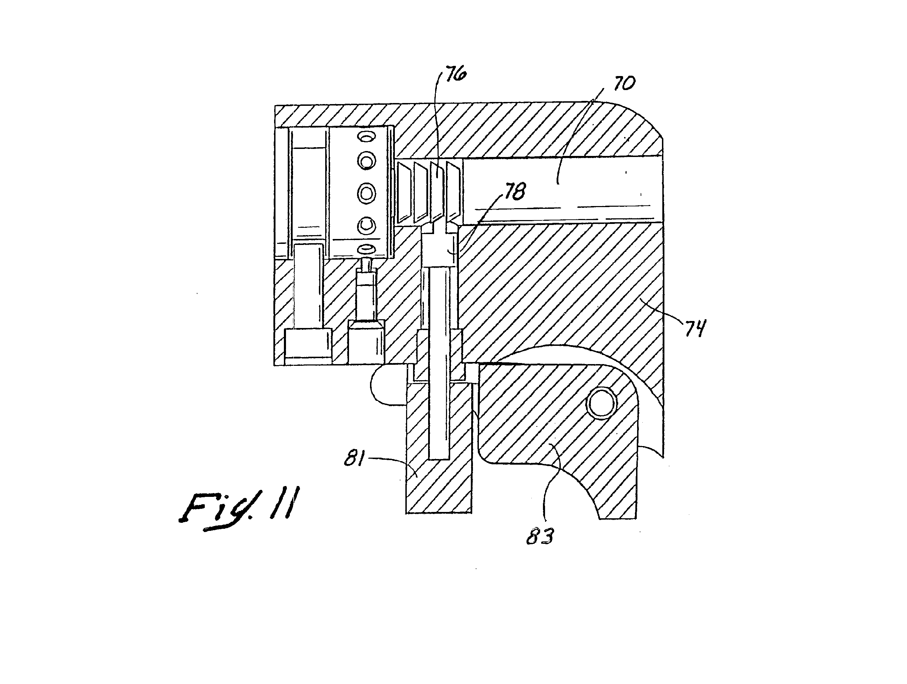

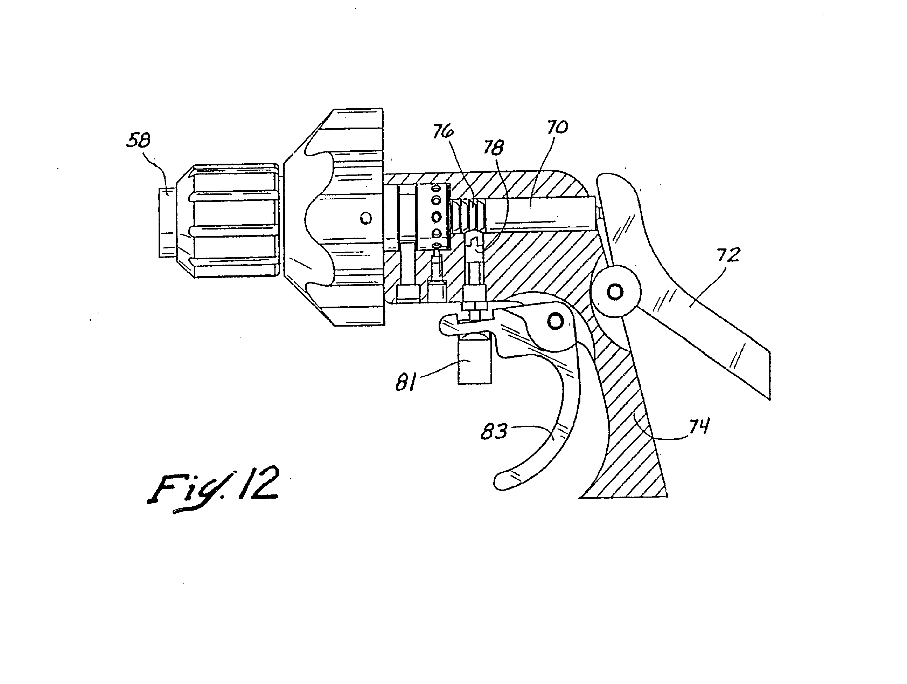

The locked ratchet assembly 30 can be released by operation of a trigger 83, which is pivotal on the handle housing 74 and operable to remove the pawl 78 from the ratchet teeth 76, as illustrated in Figure 12. With the pawl 78 biased to the active position, release of the trigger 83 permits the pawl to re-engage the ratchet teeth 76 in the active state, illustrated in Figures 9-11.

Permanent removal of the pawl 78 from the ratchet teeth 76 can be achieved by operation of the thumbscrew 81. By turning this screw 90, the pawl 78 is held in a space relationship with the ratchet teeth 76. Thus, the ratchet mechanism 30 can be permanently deactivated without the repeated operation of the trigger 83.

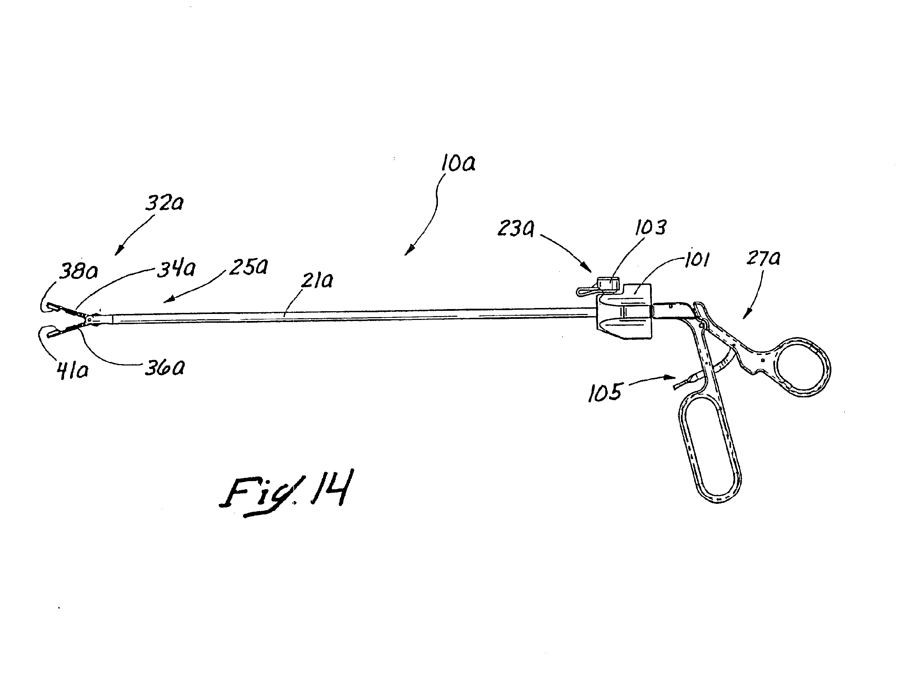

A further embodiment ofthe invention is illustrated in Figures 14-19. In this embodiment, elements of structure that are similar to those previously discussed will be designated with the same reference numeral, followed by the lower-case letter "a". Accordingly, Figure 14 illustrates a grasper 10a and a barrel 21a with a jaw assembly 32a disposed at a proximal end 23a, and a handle assembly 27a disposed at a distal end 25a. In this embodiment, a rotation knob 101, with Luer fitting 103, functions in a manner similar to the apertured cylinder 63.

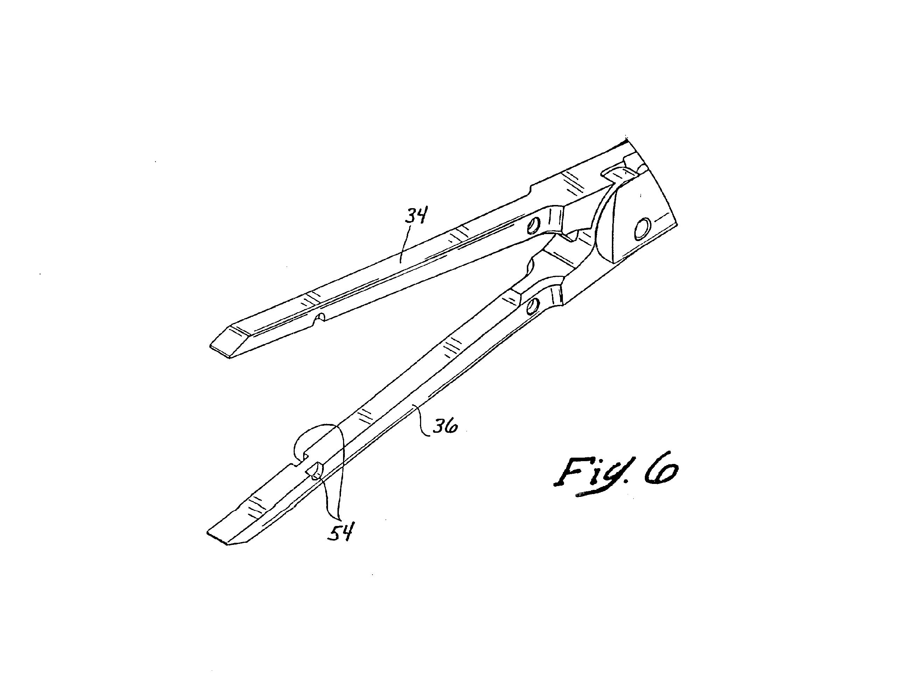

At the distal end 25, a pair of jaw inserts 38a and 41a can be mounted on associated opposing jaws 34a and 36a in a manner similar to that previously discussed with reference to Figures 5-7. A ratchet mechanism 105 is operable with the handle assembly 27a as described in greater detail below.

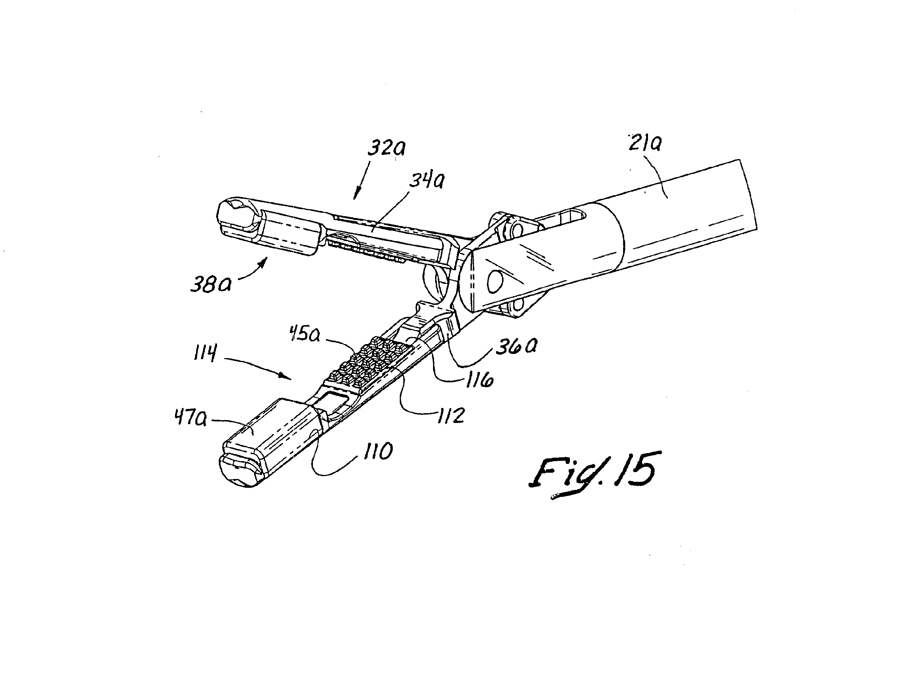

The jaw assembly 32a is described with reference to Figure 15, where the axis of the barrel 21a is designated by the reference numeral 107. In this view, the injection-molded plastic insert 41a is illustrated with an insert-molded, low-profile elastomeric pad 45a and a compression-molded, high-profile elastomeric pad 47a. Of particular interest is the relative height of these pads 45a and 47a.

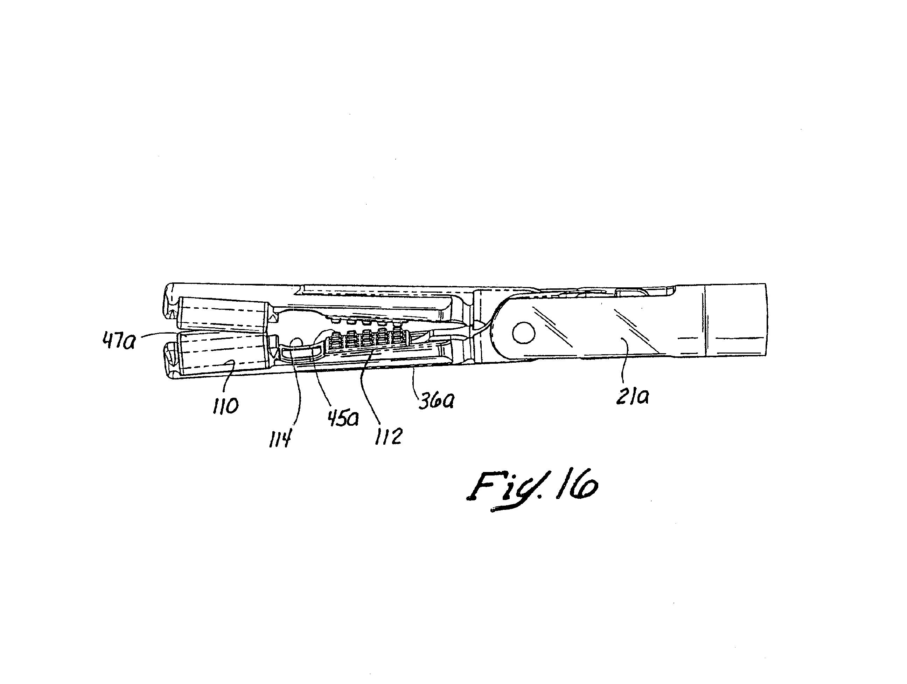

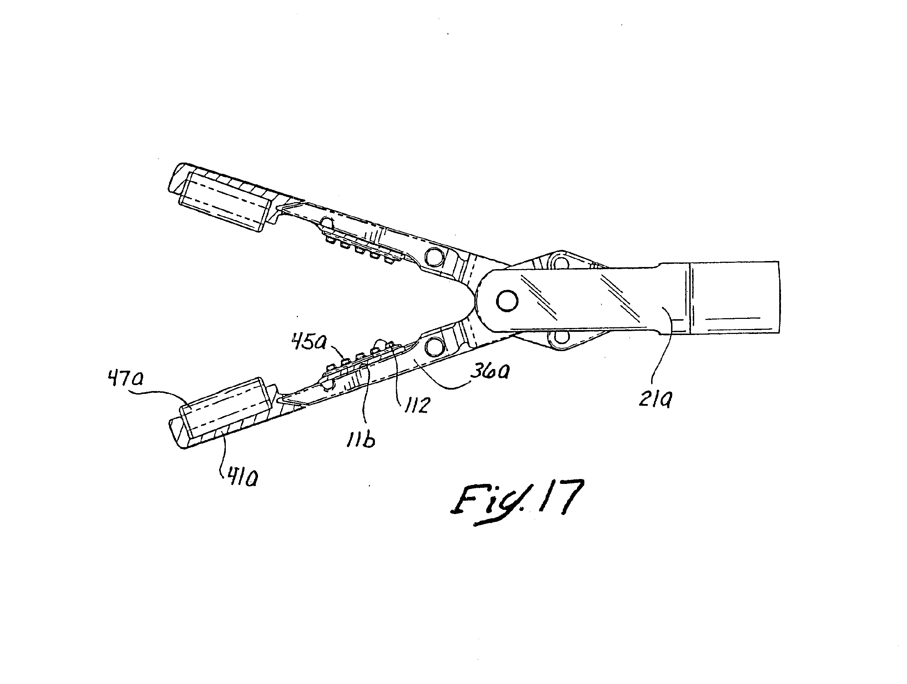

In order to enhance the grasping and capturing capability of the insert 41 a, the height of the low-profile pad 45a is minimized, while the height of the high-profile pad 47a is maximized. In addition, a surface 109, supporting the high-profile pad 47a, is lower or deeper into the jaw 36a than a surface 112, which supports the low-profile pad 45a. These surfaces 110 and 112, as well as a cutout 114 disposed therebetween, are best illustrated in the side and cross-section views of Figures 16 and 17, respectively. With the surface 110 being lower or deeper into the insert base 41a, the high-profile pad 47a can be provided with a greater thickness and therefore increased resiliency relative to that of the low-profile pad 45a. This construction maximizes the atraumatic properties of the insert 41 a, while still allowing the grasper 10a to pass through a small-diameter trocar 12 (Figure 1). In addition, the cutout 114 in the insert base 41a maximizes the area of the undercut.

In this embodiment, the high-profile pad 47a is disposed distally of the tip of the supporting jaw 36a. The surface 110 supporting the high-profile pad 47a is not only lower than the surface 112 supporting the low-profile pad 45a, but also lower than a supporting surface 116 of the jaw 36a. Measured with respect to the axis 107, it can be seen that the supporting surface 116 of the jaw 36a is spaced a distance greater than the surface 112 supporting the low-profile pad 45a, and a distance less than the surface 110 supporting the high-profile pad 47a. Thus, in this embodiment, the bottom surface of the elastomeric pad 47a is below the top surface of the jaw 36a, allowing the thickness of the pad to be maximized to enhance the atraumatic properties of the insert 41 a, while retaining the strength of the jaw 36a. Nestling of the high-profile elastomeric pad 47a also allows for a more secure bond to the plastic insert base 41 a. These relative heights of the surfaces 110, 112, and 116 are best illustrated in Figure 17.

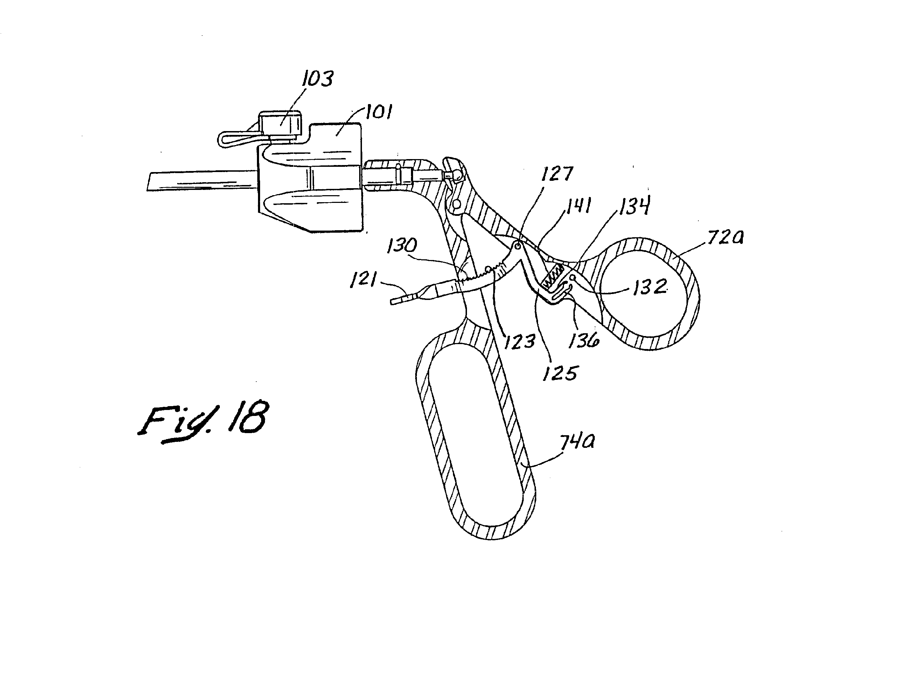

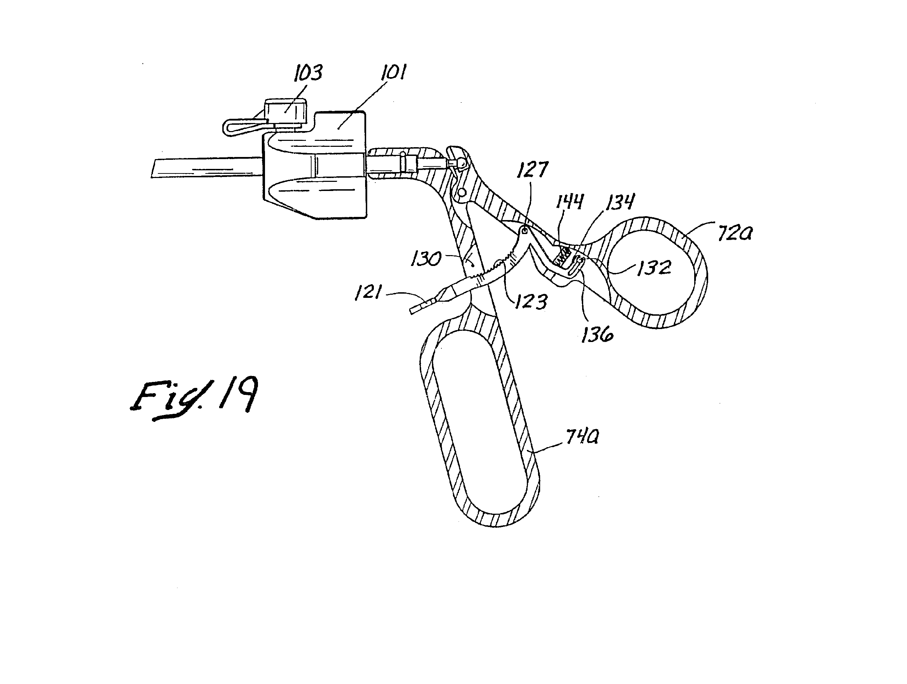

A further embodiment of the handle assembly 27a and ratchet mechanism 105 are illustrated in Figures 18 and 19. In this embodiment, the handle assembly 27a includes a non-moveable finger handle 74a and a pivotally-attached thumb handle 72a. The ratchet mechanism 105 includes a lever 121, which carries a series of ratchet teeth 123. The lever 121 includes an extension 125 and is pivotally mounted on a pin 127 fixed to the thumb handle 72a. The ratchet mechanism 105 also includes a pawl 130 fixed to the finger handle 74a.

This particular embodiment of the ratchet mechanism 105 also includes a lock pin 132, which is fixed to the thumb handle 72a, and a pair of arms 134 and 136 which are mounted on the extension 125 and pivotal with the lever 121 to engage and disengage the lock pin 132.

In operation, the lever 121 can be pivoted on the pin 127 between a first position, illustrated in Figure 18, and a second position, illustrated in Figure 19. In the first position, the lever is biased by a spring 141 so that the teeth 123 engage the pawl 130. In this position, the grasper 10a is provided with ratchet characteristics, meaning that the jaws 134a and 136a are locked into progressively closed positions as the thumb handle 72a is moved into proximity with the finger handle 74a. In order to disengage the ratchet mechanism 105, the lever 121 can be pivoted on the pin 127 against the bias of the spring 141 until the teeth 123 disengage the pawl 130.

In the second position, illustrated in Figure 19, the lever 121 is pivoted away from the pawl 130 until the arms 134 and 136 engage the lock pin 132. In this position, the ratchet teeth 123 are permanently removed from the pawl 130 until the lever 121 is manually activated to remove the arms 134, 136 from the pin 132. Once the arms 134, 136 are unlocked from the pin 132, the spring 141 can bias the lever 121 back in the first position.

It will be noted that when the lever 121 is in the second position, the ratchet characteristics are inhibited, meaning that the handle 72a, 74a can be freely moved without locking the jaws 34a and 36a to the progressively closed positions. With the ratchet lever 121 in the second position, the grasper 10a can be easily "run" without interference from the ratchet mechanism 105.

It will be understood that many other modifications can be made to the various disclosed embodiments without departing from the spirit and scope of the concept. For example, various sizes of the surgical device are contemplated as well as various types of constructions and materials. It will also be apparent that many modifications can be made to the configuration of parts as well as their interaction. For these reasons, the above description should not be construed as limiting the invention, but should be interpreted as merely exemplary of preferred embodiments. Those skilled in the art will envision other modifications within the scope and spirit of the present invention as defined by the following claims.

## Claims

1. A surgical grasper, comprising:
a pair of jaws moveable between an open position and a closed position;
a handle assembly moveable between a first position and a second position to move the jaws between the open position and the closed position;
a first resilient pad having a first profile and being disposed at the distal end of the insert;
a second resilient pad having a second profile and being disposed on the insert proximally of the first pad; and
the first profile of the first pad being higher than the second profile of the second pad.

2. The surgical grasper recited in Claim 1, further comprising:
a base forming an insert with at least one of the first resilient pad and the second resilient pad, the insert being removably mountable on an associated one of the jaws.

3. The surgical grasper recited in Claim 2, further comprising:
a linkage connected between the handle assembly and the jaws;
a ratchet assembly included in the linkage and having a series of ratchet teeth and a ratchet pawl engageable to provide the linkage with ratchet characteristics; and
a lock for disengaging the series of ratchet teeth from the ratchet pawl to inhibit the ratchet characteristics in the linkage.

4. The surgical grasper recited in Claim 3, wherein the lock comprises:
a post carried by the handle assembly; and
a pair of arms carried by one of the series of ratchet teeth and the pawl, for releasably engaging the post to lock the series of ratchet teeth and the pawl in a space relationship to inhibit the ratchet characteristics in the linkage.
